# FASCICULE DE BREVET EUROPEEN

(11) **EP 1 558 612 B1**
(45) Date de publication et mention de la délivrance du brevet: **23.08.2006**
(21) Numéro de dépôt: 03767889.3
(22) Date de dépôt: 04.11.2003
(51) Int. Cl.: C07D 471/04, A61K 31/44, C07D 235/00, C07D 221/00

(54) **DERIVES D'IMIDAZOPYRIDINE, LEUR PROCEDE DE PREPARATION ET LES COMPOSITIONS PHARMACEUTIQUES QUI LES CONTIENNENT**
IMIDAZOPYRIN-DERIVATE, VERFAHREN ZU IHRER HERSTELLUNG AND SIE ENTHALTENDE PHARMAZEUTISCHE ZUSAMENSETZUNGEN
IMIDAZOPYRIDINE DERIVATIVES, PREPARATION METHOD AND PHARMACEUTICAL COMPOSITIONS CONTAINING SAME

(30) Priorité: 05.11.2002 FR 0213802
(43) Date de publication de la demande: 03.08.2005
(73) Titulaire: Les Laboratoires Servier, 92415 Courbevoie Cédex (FR)
(72) Inventeur: RAULT, Sylvain, F-14370 Moult (FR); LANCELOT, Jean-Charles, F-14400 Le Bourg (FR); KOPP, Marina, F-14000 Caen (FR); CAIGNARD, Daniel-Henri, F-78230 Le Pecq (FR); PFEIFFER, Bruno, F-95320 Saint Leu la Forêt (FR); RENARD, Pierre, F-78150 Le Chesnay (FR); BIZOT-ESPIARD, Jean-Guy, F-75015 Paris (FR)
(86) Numéro de dépôt international: PCT/FR2003/003277
(87) Numéro de publication internationale: WO 2004/043957

(56) Documents cités:
- EP-A- 0 638 568
- WO-A-00/12089
- WO-A-98/25921
- US-A1- 2002 082 425
- OGUCHI, MINORU ET AL: "Molecular Design, Synthesis, and Hypoglycemic Activity of a Series of Thiazolidine-2,4-diones" JOURNAL OF MEDICINAL CHEMISTRY (2000), 43(16), 3052-3066, XP002242679

## Description

Du point de vue structural, de très nombreux exemples de dérivés d'imidazopyridine sont connus dans la littérature particulièrement pour leurs qualités thérapeutiques. A titre d'exemple, certains dérivés sont utilisés dans le traitement des troubles du système nerveux central (WO 0153263), des infections virales (WO 0100611), des allergies (EP 144101) ou des cancers (WO 0244156).

La protéine kinase activée par l'AMP (AMPK) est une protéine kinase impliquée dans la réponse cellulaire du stress énergétique. Cette protéine est activée par une augmentation des concentrations intra-cellulaire d'AMP consécutive à une diminution de la concentration d'ATP , lors d'un exercice physique par exemple.
L'AMPK phosphoryle et modifie l'activité d'enzymes-clé du métabolisme glucidolipidique. En effet, l'AMPK joue un rôle important dans la lipogénèse puisqu'elle inhibe la synthèse des acides gras et du cholestérol par inactivation de l'acétyl CoA Carboxylase et de l'HMGCoréductase. L'AMPK diminue l'expression de la Fatty Acid Synthase (FAS) qui contrôle la synthèse des triglycérides.
Par ailleurs, l'AMPK diminue également l'expression d'un des enzymes clés de la néoglucogénèse (PEPCK) ce qui se traduit par une inhibition de la production hépatique de glucose.
Enfin, l'AMPK augmente l'utilisation du glucose en facilitant le transport du glucose dans le muscle.
Toutes ces propriétés concourent à faire de l'AMPK une cible de choix dans le traitement du diabète et des désordres métaboliques qui lui sont associés et confère donc à la recherche d'activateurs pharmacologiques de l'AMPK un intérêt essentiel dans le traitement de ces pathologies [voir Winder WW and Hardie DG: AMP-activated protein kinase, a metabolic master switch: possible roles in type 2 diabetes; Am. J. Physiol., 40: E1-E10, (1999) et references citées].

La demanderesse a présentement découvert de nouveaux dérivés d'imidazopyridine de structure originale cycloalkylimidazopyridine leur conférant des propriétés d'activateur d'AMPK et plus précisément des propriétés antidiabétiques et antihyperlipidémiantes.

Plus particulièrement, la présente invention concerne les composés de formule (I) : 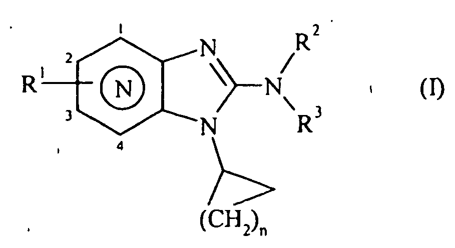 dans laquelle :
◆ R¹ représente un atome d'hydrogène, d'halogène, un groupement alkyle, polyhalogénoalkyle, cyano, nitro, hydroxycarbonyle, alkoxycarbonyle, aminocarbonyle, alkylaminocarbonyle, ou dialkylaminocarbonyle,
◆ R² représente un atome d'hydrogène, un groupement alkyle, aryle éventuellement substitué, hétéroaryle éventuellement substitué ou un groupement R²⁰-C(X)- avec :
   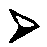 R²⁰ représentant un groupement alkyle, alkoxy, amino, alkylamino, dialkylamino, aryle éventuellement substitué ou hétéroaryle éventuellement substitué,
   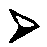 X représentant un atome d'oxygène, de soufre, ou un groupement NR²¹ où R²¹ représente un atome d'hydrogène ou un groupement alkyle,
◆ R³ représente un atome d'hydrogène, ou un groupement alkyle,
◆ n représente un entier compris inclusivement entre 1 et 6,
◆ la représentation 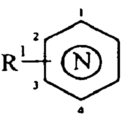 signifie : 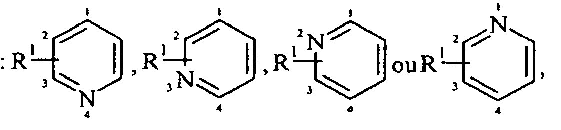 leurs énantiomères, diastéréoisomères, ainsi que leurs sels d'addition à un acide ou à une base pharmaceutiquement acceptable,
étant entendu que :
- le terme "alkyle" désigne une chaîne hydrocarbonée, linéaire ou ramifiée, contenant de 1 à 6 atomes de carbone,
- le terme "alkoxy" désigne un groupement alkyle-oxy dont la chaîne alkyle, linéaire ou ramifiée, contient de 1 à 6 atomes de carbone,
- le terme "aryle" désigne un groupement phényle ou biphényle,
- le terme "polyhalogénoalkyle" désigne une chaîne carbonée, linéaire ou ramifiée, contenant de 1 à 3 atomes de carbone et de 1 à 7 atomes d'halogène,
- le terme "hétéroaryle" désigne un groupement de 5 à 11 chaînons, monocyclique ou bicyclique, dans lequel au moins un des cycles est aromatique, comportant dans le monocycle ou dans le bicycle 1, 2 ou 3 hétéroatomes, choisis parmi l'azote, l'oxygène et le soufre, et
- le terme "éventuellement substitué" associé aux expressions aryle et hétéroaryle signifie que les groupements concernés sont éventuellement substitués par un ou deux substituants, identiques ou différents, choisis parmi les atomes d'halogène et les groupements alkyle, alkoxy, polyhalogénoalkyle, hydroxy, cyano, nitro, amino (éventuellement substitué par un ou deux groupements alkyle) et -C(O)R_{d} avec R_{d} représentant un groupement choisi parmi hydroxy, alkoxy et amino, étant entendu que le groupement hétéroaryle peut être en plus substitué par un groupement oxo sur la partie non-aromatique de l'hétéroaryle.

Parmi les acides pharmaceutiquement acceptables, on peut citer à titre non limitatif, les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif, l'hydroxyde de sodium, l'hydroxyde de potassium, la triéthylamine, etc...

La représentation préférée de 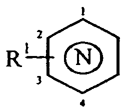 est

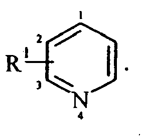

Un aspect avantageux de l'invention concerne les composés pour lesquels R¹ représente un atome d'hydrogène.

Des composés préférés de l'invention sont ceux pour lesquels R² représente un atome d'hydrogène ou un groupement R²⁰-C(O)-.

Un autre aspect avantageux concerne les composés de formule (I) pour lesquels R³ représente un atome d'hydrogène.

Le groupement R²⁰ préféré de l'invention est le groupement alkoxy et plus particulièrement le groupement éthoxy.

Dans les composés préférés de l'invention, n représente un entier égal à 4, 5 ou 6 et plus particulièrement 5.

Un aspect particulièrement avantageux de l'invention concerne les composés de formule (I) pour lesquels R¹ représente un atome d'hydrogène, R² représente un atome d'hydrogène, ou un groupement R²⁰-C(O)- avec R²⁰ représentant un groupement alkoxy, et n est égal à 4 ou 5.

Parmi les composés préférés de l'invention, on peut citer plus particulièrement la 3-cycloheptyl-3*H*-imidazo[4,5-*b*]pyridin-2-amine et la 3-cyclooctyl-3*H*-imidazo[4,5-*b*]pyridin-2-amine.

L'invention concerne également le procédé de préparation des composés de formule (I), caractérisés en ce que l'on utilise comme produit de départ les composés de formule (II) : 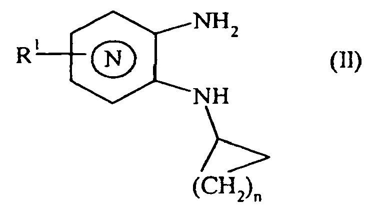 dans laquelle R¹ et n sont tels que définis dans la formule (I), composés de formule (II) qui se condensent aux dérivés d'isothiocyanate (III) :

S=C=N-C(X)-R²⁰ (III)

dans laquelle X et R²⁰ sont tels que définis dans la formule (I), pour conduire aux intermédiaires de formule (IV) : 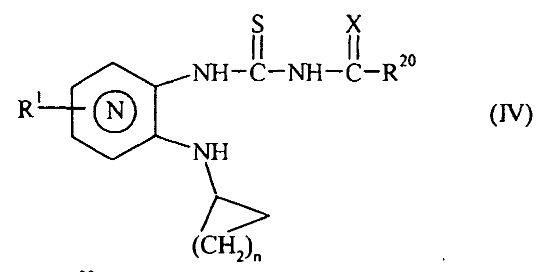 dans laquelle R¹, n, X et R²⁰ sont tels que définis dans la formule (I),
composés de formule (IV) qui subissent une cyclisation intramoléculaire en milieu basique et en présence de catalyseur approprié pour conduire aux composés (I/a) : 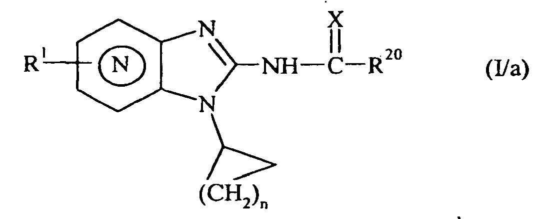 cas particuliers des composés de formule (I) pour lesquels R¹, n, X et R²⁰ sont tels que définis dans la formule (I),
composés de formule (I/a) qui en milieu acide se transforment éventuellement en composés de formule (I/b) : 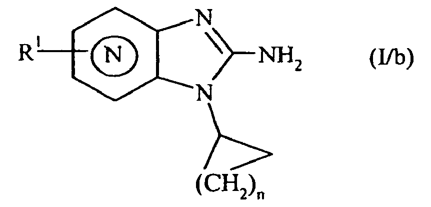 cas particuliers des composés de formule (I) pour lesquels R¹ et n sont tels que définis dans la formule (I),
composés de formule (I/b) dont la fonction amine peut être fonctionnalisée en milieu basique, à l'aide d'halogénure d'alkyle Alk-Z (avec Alk représentant un groupement alkyle et Z un atome d'halogène) pour conduire aux composés de formule (I/c) : 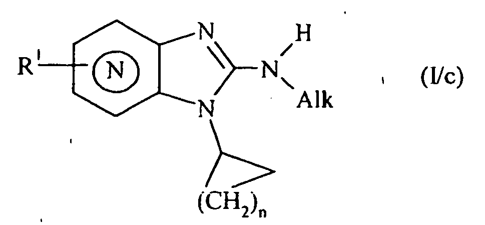 cas particulier des composés de formule (I) dans lesquels R¹ et n sont tels que définis dans la formule (I) et Alk est tel que défini précédemment,
composés de formule (I/b) et (I/c) qui peuvent, en milieu basique, éventuellement en présence de catalyseurs appropriés, réagir avec R² - Z' (avec R² tel que défini dans la formule (I) et Z' représentant un groupement nucléofuge comme un atome d'halogène ou un groupement trihalogénoalkyle) pour conduire aux composés de formule (I),
composés (I/a), (I/b) et (I/c) formant la totalité des composés de formule (I), et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable,
étant entendu :
- qu'à tout moment jugé opportun au cours du procédé précédemment décrit, le ou les groupements carbonyle, amino, alkylamino des réactifs de départ (II) et (III) peuvent être protégés puis, après condensation, déprotégés pour les besoins de la synthèse,
- que les réactifs (II), et (III) sont préparés selon des modes opératoires connus, décrits dans la littérature.

Les composés montrent notamment une excellente activité dans la réduction des taux triglycérides et de glucose sanguin. Ces propriétés justifient leur application en thérapeutique dans le traitement et/ou la prophylaxie des hyperglycémies, dyslipidémies, et plus particulièrement dans le traitement des diabètes non insulino dépendants de type II, de l'obésité, de l'intolérance au glucose, et des complications diabétiques en particulier au niveau cardiovasculaire.

L'activité de ces composés est également recommandée pour le traitement et/ou la prophylaxie d'autres maladies incluant le diabète de type I, les hypertriglycéridémies, le syndrome métabolique, la résistance à l'insuline, les dyslipidémies chez le diabétique, les hyperlipidémies et l'hypercholestérolémie.

La présente invention a également pour objet les compositions pharmaceutiques renfermant comme principe actif au moins un composé de formule (I) seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.

Les exemples suivants illustrent l'invention et ne la limitent en aucune façon. Les structures des composés décrits ont été confirmées par des techniques spectroscopiques et spectrométriques usuelles.

Les produits de départ utilisés sont des produits connus ou préparés selon des modes opératoires connus.

### PREPARATION 1 : N²-Cyclohexyl-2,3-pyridinediamine

### Stade a : N-Cyclohexyl-3-nitro-2-pyridinamine

Un mélange constitué de 0,1 mole (15,85 g) de 2-chloro-3-nitropyridine et de 0,1 mole (11,50 ml) de cyclohexylamine est chauffé à 120°C pendant 4 heures dans 250 ml DMF en présence de carbonate de potassium (13,81 g). La solution est ensuite extraite par 200 ml d'éther et la phase organique est lavée trois fois à l'eau. Après séchage sur sulfate de magnésium, l'éther est évaporé.

### Stade b : N²-Cyclohexyl-2,3-pyridinediamine

On introduit dans l'autoclave 800 ml d'éthanol, 0,05 mole (11,06 g) du dérivé nitré préparé au stade précédent et 3,5 g de charbon palladié. Le mélange est chauffé à 60°C pendant 30 mn sous 50 kg d'hydrogène, puis agité horizontalement à température ambiante pendant 3 heures pour homogénéiser la solution. Après ce délai, la solution est filtrée sur büchner, puis sur double filtre pour éliminer les derniers résidus de charbon palladié et l'éthanol est évaporé sous pression réduite.

### PREPARATION 2 : N²-Cycloheptyl-2,3-pyridinediamine

Le protocole expérimental est identique à celui utilisé dans la préparation 1, en partant au stade a de la cycloheptylamine à la place de la cyclohexylamine.

### PREPARATION 3 : N²-Cyclooctyl-2,3-pyridinediamine

Le protocole expérimental est identique à celui utilisé dans la préparation 1, en partant au stade a de la cyclooctylamine à la place de la cyclohexylamine.

### EXEMPLE 1 : 3-Cyclohexyl-3H-imidazo[4,5-b]pyridin-2-ylcarbamate d'éthyle

### Stade a : {[2-(Cyclohexylamino)-3-pyridinyl]imino}(diéthylamino)méthylcarbamate d'éthyle

Un mélange de 0,02 mole (3,82 g) de 3-amino-2-cyclohexylaminopyridine de la préparation 1 et de 0,02 mole d'isothiocyanate d'éthoxycarbonyle est agité dans 50 ml de DMF à température ambiante pendant 3 heures. La solution est alors refroidie à 0°C et 0,05 mole de dipropylamine puis 0,02 mole de chlorure mercurique sont ajoutés successivement. Au bout de 15 minutes, le bain de glace est enlevé et la solution est agitée à température ambiante pendant 4 heures. Après avoir dilué la solution par 100 ml d'acétate d'éthyle, on la filtre sur célite et les solvants sont évaporés sous pression réduite. Le produit brut obtenu est ensuite recristallisé dans l'acétonitrile.

### Stade b : 3-Cyclohexyl-3H-imidazo[4,5-b]pyridin-2-ylcarbamate d'éthyle

On dissout 0,0048 mole du composé préparé au stade précédent dans 100 ml d'une solution de méthanol et de soude à 15 % (50/50). Après avoir chauffé la solution à reflux pendant 3 heures, le méthanol est évaporé. Le précipité ainsi obtenu est essoré, lavé à l'eau et recristallisé dans l'acétonitrile.
Point de fusion : 264°C

### EXEMPLE 2 : 3-Cycloheptyl-3H-imidazo[4,5-b]pyridin-2-ylcarbamate d'éthyle

Le protocole expérimental est identique à celui utilisé dans l'exemple 1, en partant au stade a de la 3-amino-2-cyclohephylaminopyridine de la préparation 2, à la place de la 3-amino-2-cyclohexylaminopyridine.
Point de fusion : 177°C

### EXEMPLE 3 : 3-Cyclohexyl-3H-imidazo[4,5-b]pyridin-2-amine

On additionne le composé de l'exemple 1 à 100 ml d'une solution de dioxane saturée en acide chlorhydrique gazeux et on chauffe la solution pendant 12 heures à reflux. Après refroidissement de la solution, le précipité est essoré, lavé au bicarbonate de soude puis recristallisé dans l'acétonitrile.
Point de fusion : 210°C

### EXEMPLE 4 : 3-Cycloheptyl-3H-imidazo[4,5-b]pyridin-2-amine

Le protocole expérimental est identique à celui utilisé dans l'exemple 3 en utilisant comme produit de départ le composé de l'exemple 2.
Point de fusion : 210°C

### EXEMPLE 5 : 3-Cyclooctyl-3H-imidazo[4,5-b]pyridin-2-amine

Le protocole expérimental est identique à celui utilisé dans l'exemple 1, en partant au stade a de la 3-amino-2-cyclooctylaminopyridine de la préparation 3, à la place de la 3-amino-2-cyclohexylaminopyridine.
Point de fusion : 198°C

### ETUDE PHARMACOLOGIQUE

### EXEMPLE A : Activité de l'AMPK dans un modèle cellulaire : hépatocytes isolés de rat

Les hépatocytes sont isolés selon la technique de Berry et Friend [J. Cell Biol, 43, 506-520 (1969)]. L'activité de l'AMPK a été mesurée selon la méthode décrite par Davies et al. [Eur. J. Biochem., 186, 123-128 (1989)]. Celle-ci implique la phosphorylation à partir de [γ-³²P]-ATP d'un substrat peptidique (SAMS), basé sur la séquence entourant le site phosphorylé par l'AMPK de l'ACC. La réaction de mesure de l'activité de l'AMPK se termine par le dépôt d'un aliquot du milieu réactionnel sur un papier de phosphocellulose (Whatman P81), sur lequel le peptide SAMS se fixe et dont la radioactivité est mesurée après lavage.

A titre d'exemple, le composé de l'Exemple 4 active l'AMPK, après 30 minutes à la concentration de 500 µM, de 312 % (vs. la valeur basale) alors qu'à la même concentration dans les mêmes conditions l'AICARiboside, pris pour référence, l'active de 178 %.

### EXEMPLE B : Activité hypolipémiante

Les produits de l'invention ont été testés *in vivo* chez la souris obèse ob/ob, utilisé comme modèle d'insulinorésistance associée à l'obésité. A titre d'exemple, le composé de l'Exemple 4 baisse significativement les triglycérides à 125 mg/kg per os, alors qu'avec la metformine, la même diminution est obtenue à 250 mg/kg per os.
Dans ce modèle, les composés de l'invention se sont donc révélés être de puissants hypolipémiants.

### EXEMPLE C : Composition pharmaceutique

| Formule de préparation pour 1000 comprimés dosés à 5 mg : | |
|---|---|
| Composé de l'exemple 4 | 5 g |
| Hydroxypropylméthylcellulose | 2 g |
| Amidon de blé | 10 g |
| Lactose | 100 g |
| Stéarate de magnésium | 3 g |

## Revendications

1. Composés de formule (I) : 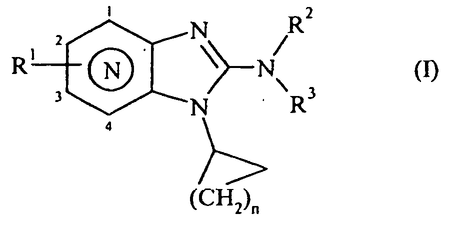 dans laquelle :
◆ R¹ représente un atome d'hydrogène, d'halogène, un groupement alkyle, polyhalogénoalkyle, cyano, nitro, hydroxycarbonyle, alkoxycarbonyle, aminocarbonyle, alkylaminocarbonyle, ou dialkylaminocarbonyle,
◆ R² représente un atome d'hydrogène, un groupement alkyle, aryle éventuellement substitué, hétéroaryle éventuellement substitué ou un groupement R²⁰-C(X)- avec :
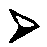 R²⁰ représentant un groupement alkyle, alkoxy, amino, alkylamino, dialkylamino, aryle éventuellement substitué ou hétéroaryle éventuellement substitué,
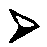 X représentant un atome d'oxygène, de soufre, ou un groupement NR²¹ où R²¹ représente un atome d'hydrogène ou un groupement alkyle,
◆ R³ représente un atome d'hydrogène, ou un groupement alkyle,
◆ n représente un entier compris inclusivement entre 1 et 6,
◆ la représentation 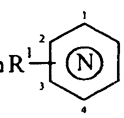 signifie: 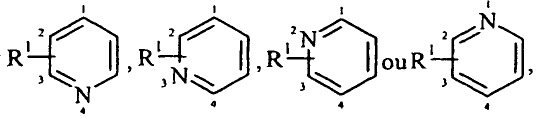 leurs énantiomères, diastéréoisomères, ainsi que leurs sels d'addition à un acide ou à une base pharmaceutiquement acceptable,
étant entendu que :
- le terme "alkyle" désigne une chaîne hydrocarbonée, linéaire ou ramifiée, contenant de 1 à 6 atomes de carbone,
- le terme "alkoxy" désigne un groupement alkyle-oxy dont la chaîne alkyle, linéaire ou ramifiée, contient de 1 à 6 atomes de carbone,
- le terme "aryle" désigne un groupement phényle ou biphényle,
- le terme "polyhalogénoalkyle" désigne une chaîne carbonée, linéaire ou ramifiée, contenant de 1 à 3 atomes de carbone et de 1 à 7 atomes d'halogène,
- le terme "hétéroaryle" désigne un groupement de 5 à 11 chaînons, monocyclique ou bicyclique, dans lequel au moins un des cycles est aromatique, comportant dans le monocycle ou dans le bicycle 1, 2 ou 3 hétéroatomes, choisis parmi l'azote, l'oxygène et le soufre, et
- le terme "éventuellement substitué" associé aux expressions aryle et hétéroaryle signifie que les groupements concernés sont éventuellement substitués par un ou deux substituants, identiques ou différents, choisis parmi les atomes d'halogène et les groupements alkyle, alkoxy, polyhalogénoalkyle, hydroxy, cyano, nitro, amino (éventuellement substitué par un ou deux groupements alkyle) et -C(O)R_{d} avec R_{d} représentant un groupement choisi parmi hydroxy, alkoxy et amino, étant entendu que le groupement hétéroaryle peut être en plus substitué par un groupement oxo sur la partie non-aromatique de l'hétéroaryle.

2. Composés de formule (I) selon la revendication 1 pour lesquels la représentation 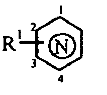 correspond à 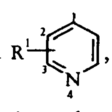 leurs énantiomères, diastéréoisomères, ainsi que leurs sels d'addition à un acide ou à une base pharmaceutiquement acceptable.

3. Composés de formule (I) selon l'une quelconque des revendications 1 ou 2 pour lesquels R¹ représente un atome d'hydrogène, leurs énantiomères, diastéréoisomères, ainsi que leurs sels d'addition à un acide ou à une base pharmaceutiquement acceptable.

4. Composés de formule (I) selon l'une quelconque des revendications 1 à 3 pour lesquels R² représente un atome d'hydrogène, leurs énantiomères, diastéréoisomères, ainsi que leurs sels d'addition à un acide ou à une base pharmaceutiquement acceptable.

5. Composés de formule (1) selon l'une quelconque des revendications 1 à 3 pour lesquels R² représente un groupement R²⁰-C(O)-, leurs énantiomères, diastéréoisomères, ainsi que leurs sels d'addition à un acide ou à une base pharmaceutiquement acceptable.

6. Composés de formule (I) selon l'une quelconque des revendications 1 à 5 pour lesquels R³ représente un atome d'hydrogène, leurs énantiomères, diastéréoisomères, ainsi que leurs sels d'addition à un acide ou à une base pharmaceutiquement acceptable.

7. Composés de formule (I) selon l'une quelconque des revendications 1 à 3, 5 ou 6 pour lesquels R²⁰ représente un groupement alkoxy, leurs énantiomères, diastéréoisomères, ainsi que leurs sels d'addition à un acide ou à une base pharmaceutiquement acceptable.

8. Composés de formule (I) selon l'une quelconque des revendications 1 à 7 pour lesquels n représente un entier compris inclusivement entre 4 et 6, leurs énantiomères, diastéréoisomères, ainsi que leurs sels d'addition à un acide ou à une base pharmaceutiquement acceptable.

9. Composés de formule (1) selon l'une quelconque des revendications 1 à 8 pour lesquels 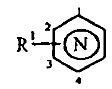 représente 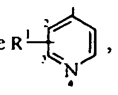 R¹ représente un atome d'hydrogène, R² représente un atome d'hydrogène ou un groupement R²⁰-C(O)- avec R²⁰ représentant un groupement alkoxy et n égal à 4 ou 5, leurs énantiomères, diastéréoisomères, ainsi que leurs sels d'addition à un acide ou à une base pharmaceutiquement acceptable.

10. Composés de formule (1) selon l'une quelconque des revendications 1 à 4, 6, 8 ou 9 qui est la 3-cycloheptyl-3*H*-imidazo[4,5-*b*]pyridin-2-amine.

11. Composés de formule (I) selon l'une quelconque des revendications 1 à 4, 6 ou 8 qui est la 3-cyclooctyl-3*H*-imidazo[4,5-*b*]pyridin-2-amine.

12. Procédé de préparation des composés de formule (1) selon la revendication 1,
**caractérisé en ce que** l'on utilise comme produit de départ un composé de formule (II) : 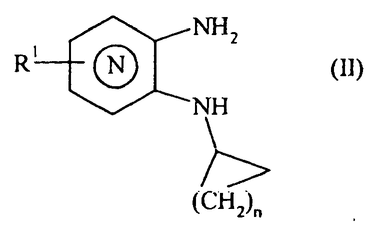 dans laquelle R¹ et n sont tels que définis dans la formule (I), composés de formule (II) qui se condensent aux dérivés d'isothiocyanate (III) :
S = C = N - C(X) -R²⁰ (III)
dans laquelle X et R²⁰ sont tels que définis dans la formule (I), pour conduire aux intermédiaires de formule (IV) : 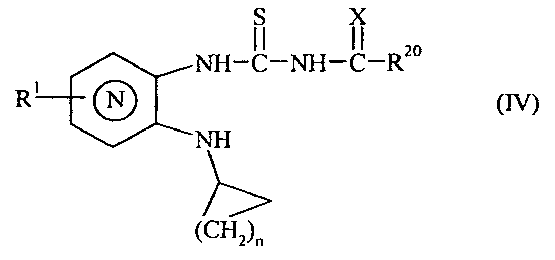 dans laquelle R¹, n, X et R²⁰ sont tels que définis dans la formule (I),
composés de formule (IV) qui subissent une cyclisation intramoléculaire en milieu basique et en présence de catalyseur approprié pour conduire aux composés (I/a) : 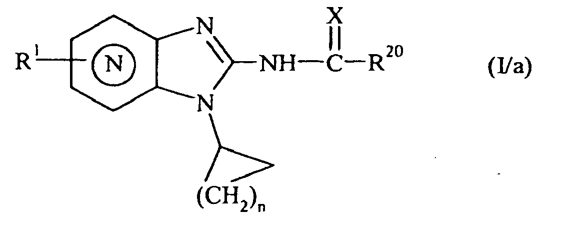 cas particuliers des composés de formule (I) pour lesquels R¹, n, X et R²⁰ sont tels que définis dans la formule (I),
composés de formule (I/a) qui en milieu acide se transforment éventuellement en composés de formule (I/b) : 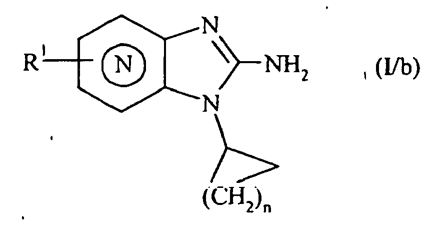 cas particuliers des composés de formule (I) pour lesquels R¹ et n sont tels que définis dans la formule (I),
composés de formule (I/b) dont la fonction amine peut être fonctionnalisée en milieu basique, à l'aide d'halogénure d'alkyle Alk-Z (avec Alk représentant un groupement alkyle et Z un atome d'halogène) pour conduire aux composés de formule (I/c) : 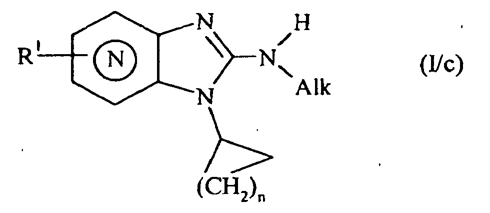 cas particulier des composés de formule (I) dans lesquels R¹ et n sont tels que définis dans la formule (I) et Alk est tel que défini précédemment,
composés de formule (I/b) et (I/c) qui peuvent, en milieu basique, éventuellement en présence de catalyseurs appropriés, réagir avec R² - Z' (avec R² tel que défini dans la formule (I) et Z' représentant un groupement nucléofuge comme un atome d'halogène ou un groupement trihalogénoalkyle) pour conduire aux composés de formule (I),
composés (I/a), (I/b) et (I/c) formant la totalité des composés de formule (I), et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable, étant entendu :
- qu'à tout moment jugé opportun au cours du procédé précédemment décrit, le ou les groupements carbonyle, amino, alkylamino des réactifs de départ (II) et (III) peuvent être protégés puis, après condensation, déprotégés pour les besoins de la synthèse,
- que les réactifs (II), et (III) sont préparés selon des modes opératoires connus, décrits dans la littérature.

13. Composition pharmaceutique contenant comme principe actif au moins un composé selon l'une quelconque des revendications 1 à 11, seul ou en combinaison avec un ou plusieurs excipients ou véhicules inertes, non toxiques et pharmaceutiquement acceptable.

14. Composition pharmaceutique selon la revendication 13 contenant au moins un principe actif selon l'une quelconque des revendications 1 à 11 utile pour la fabrication dé médicaments utiles comme activateur d'AMPK.

15. Composition pharmaceutique selon la revendication 13 contenant au moins un principe actif selon l'une quelconque des revendications 1 à 11 utiles pour la fabrication de médicaments traitant le diabète de type II non insulinodépendant, l'obésité, le diabète de type I, l'hyperlipidémie, l'hypercholestérolémie et leurs complications cardiovasculaires.

16. Composition pharmaceutique selon la revendication 13 contenant au moins un principe actif selon l'une quelconque des revendications 1 à 11 utiles pour la fabrication de médicaments traitant le diabète de type I, II et ses complications cardiovasculaires.

17. Composition pharmaceutique selon la revendication 13 contenant au moins un principe actif selon l'une quelconque des revendications 1 à 11 utiles pour la fabrication de médicaments traitant le diabète de type I et II.

## Claims

1. Compounds of formula (I): 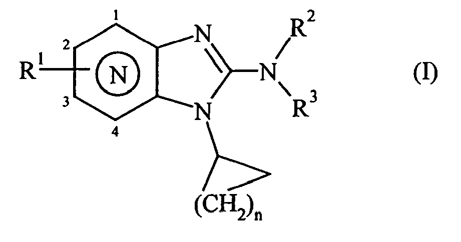 wherein:
◆ R¹ represents a hydrogen atom, a halogen atom or an alkyl, polyhaloalkyl, cyano, nitro, hydroxycarbonyl, alkoxycarbonyl, aminocarbonyl, alkylaminocarbonyl or dialkylaminocarbonyl group,
◆ R² represents a hydrogen atom, an alkyl group, an optionally substituted aryl group, an optionally substituted heteroaryl group, or a group R²⁰-C(X)- wherein:
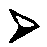 R²⁰ represents an alkyl group, an alkoxy group, an amino group, an alkylamino group, a dialkylamino group, an optionally substituted aryl group or an optionally substituted heteroaryl group,
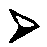 X represents an oxygen atom, a sulphur atom, or a group NR²¹ wherein R²¹ represents a hydrogen atom or an alkyl group,
◆ R³ represents a hydrogen atom or an alkyl group,
◆ n represents an integer from 1 to 6 inclusive,
◆ the representation 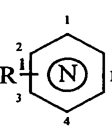 means: 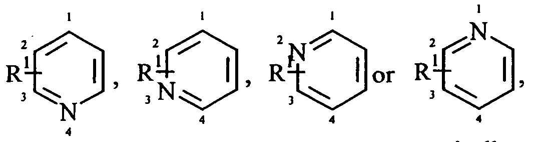 their enantiomers, diastereoisomers and also addition salts thereof with a pharmaceutically acceptable acid or base, it being understood that:
- the term "alkyl" denotes a linear or branched hydrocarbon chain containing from 1 to 6 carbon atoms,
- the term "alkoxy" denotes an alkyl-oxy group in which the alkyl chain, which is linear or branched, contains from 1 to 6 carbon atoms,
- the term "aryl" denotes a phenyl or biphenyl group,
- the term "polyhaloalkyl" denotes a linear or branched carbon chain containing from 1 to 3 carbon atoms and from 1 to 7 halogen atoms,
- the term "heteroaryl" denotes a group having from 5 to 11 ring members which is monocyclic or bicyclic, in which at least one of the rings is aromatic, and which contains in the monocyclic ring system or in the bicyclic ring system 1, 2 or 3 hetero atoms selected from nitrogen, oxygen and sulphur, and
- the expression "optionally substituted" associated with the expressions aryl and heteroaryl means that the groups in question are optionally substituted by one or two identical or different substituents selected from halogen atoms and the groups alkyl, alkoxy, polyhaloalkyl, hydroxy, cyano, nitro, amino (optionally substituted by one or two alkyl groups) and -C(O)R_{d} wherein R_{d} represents a group selected from hydroxy, alkoxy and amino, it being understood that the heteroaryl group may also be substituted by an oxo group on the non-aromatic moiety of the heteroaryl.

2. Compounds of formula (I) according to claim 1 wherein the representation 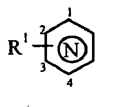 corresponds to 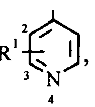 their enantiomers, diastereoisomers and also addition salts thereof with a pharmaceutically acceptable acid or base.

3. Compounds of formula (I) according to either claim 1 or claim 2 wherein R¹ represents a hydrogen atom, their enantiomers, diastereoisomers and also addition salts thereof with a pharmaceutically acceptable acid or base.

4. Compounds of formula (I) according to any one of claims 1 to 3 wherein R² represents a hydrogen atom, their enantiomers, diastereoisomers and also addition salts thereof with a pharmaceutically acceptable acid or base.

5. Compounds of formula (I) according to any one of claims 1 to 3 wherein R² represents a group R²⁰-C(O)-, their enantiomers, diastereoisomers and also addition salts thereof with a pharmaceutically acceptable acid or base.

6. Compounds of formula (I) according to any one of claims 1 to 5 wherein R³ represents a hydrogen atom, their enantiomers, diastereoisomers and also addition salts thereof with a pharmaceutically acceptable acid or base.

7. Compounds of formula (I) according to any one of claims 1 to 3, 5 or 6 wherein R²⁰ represents an alkoxy group, their enantiomers, diastereoisomers and also addition salts thereof with a pharmaceutically acceptable acid or base.

8. Compounds of formula (I) according to any one of claims 1 to 7 wherein n represents an integer from 4 to 6 inclusive, their enantiomers, diastereoisomers and also addition salts thereof with a pharmaceutically acceptable acid or base.

9. Compounds of formula (I) according to any one of claims 1 to 8 wherein 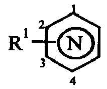 represents 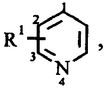 R¹ represents a hydrogen atom, R² represents a hydrogen atom or a group R²⁰-C(O)- wherein R²⁰ represents an alkoxy group, and n is 4 or 5, their enantiomers, diastereoisomers and also addition salts thereof with a pharmaceutically acceptable acid or base.

10. Compound of formula (I) according to any one of claims 1 to 4, 6, 8 or 9 which is 3-cycloheptyl-3*H*-imidazo[4,5-*b*]pyridine-2-amine.

11. Compound of formula (I) according to any one of claims 1 to 4, 6 or 8 which is 3-cyclooctyl-3*H*-imidazo[4,5-*b*]pyridine-2-amine.

12. Process for the preparation of compounds of formula (I) according to claim 1,
**characterised in that** there is used as starting material a compound of formula (II): 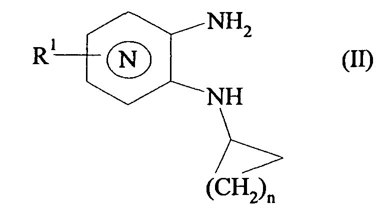 wherein R¹ and n are as defined for formula (I), which compounds of formula (II) are condensed with isothiocyanate compounds (III):
S = C = N - C(X) -R²⁰ (III)
wherein X and R²⁰ are as defined for formula (I), to yield the intermediates of formula (IV): 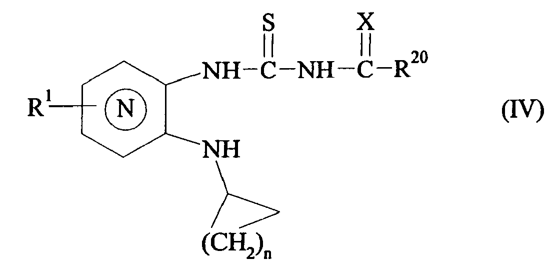 wherein R¹, n, X and R²⁰ are as defined for formula (I),
which compounds of formula (IV) undergo intramolecular cyclisation in a basic medium and in the presence of a suitable catalyst to yield the compounds (I/a): 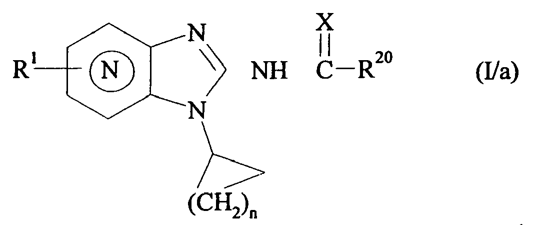 which are particular cases of the compounds of formula (I) wherein R¹, n, X and R²⁰ are as defined for formula (I),
which compounds of formula (I/a) are optionally converted, in an acid medium, into compounds of formula (I/b): 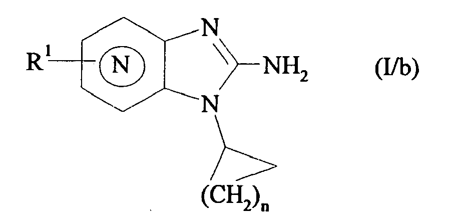 which are particular cases of the compounds of formula (I) wherein R¹ and n are as defined for formula (I),
in which compounds of formula (I/b) the amine function may be functionalised in a basic medium, with the aid of an alkyl halide Alk-Z (wherein Alk represents an alkyl group and Z represents a halogen atom), to yield the compounds of formula (I/c): 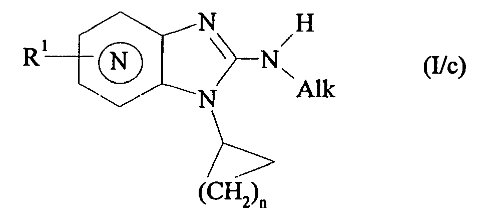 which are a particular case of the compounds of formula (I) wherein R¹ and n are as defined for formula (I) and Alk is as defined hereinbefore,
which compounds of formulae (I/b) and (I/c) may, in a basic medium, optionally in the presence of suitable catalysts, be reacted with R² - Z' (wherein R² is as defined for formula (I) and Z' represents a nucleofugal group, such as a halogen atom or a trihaloalkyl group) to yield the compounds of formula (I),
which compounds (I/a), (I/b) and (I/c) constitute the totality of the compounds of formula (I) and:
- which may, where necessary, be purified according to a conventional purification technique,
- which are separated, where necessary, into the stereoisomers according to a conventional separation technique,
- which are converted, if desired, into their addition salts with a pharmaceutically acceptable acid or base,
it being understood that:
- at any time considered to be appropriate in the course of the process described above, for the requirements of synthesis the carbonyl, amino or alkylamino group(s) of the starting reagents (II) and (III) may be protected and then, after condensation, deprotected,
- the reagents (II) and (III) are prepared according to known procedures described in the literature.

13. Pharmaceutical composition comprising as active ingredient at least one compound according to any one of claims 1 to 11, alone or in combination with one or more inert, non-toxic, pharmaceutically acceptable excipients or carriers.

14. Pharmaceutical composition according to claim 13 comprising at least one active ingredient according to any one of claims 1 to 11 for use in the manufacture of medicaments for use as AMPK activator.

15. Pharmaceutical composition according to claim 13 comprising at least one active ingredient according to any one of claims 1 to 11 for use in the manufacture of medicaments that treat non-insulin-dependent, type II diabetes, obesity, type I diabetes, hyperlipidaemia, hypercholesterolaemia and their cardiovascular complications.

16. Pharmaceutical composition according to claim 13 comprising at least one active ingredient according to any one of claims 1 to 11 for use in the manufacture of medicaments that treat type I and II diabetes and its cardiovascular complications.

17. Pharmaceutical composition according to claim 13 comprising at least one active ingredient according to any one of claims 1 to 11 for use in the manufacture of medicaments that treat type I and II diabetes.

## Patentansprüche

1. Verbindungen der Formel (I): 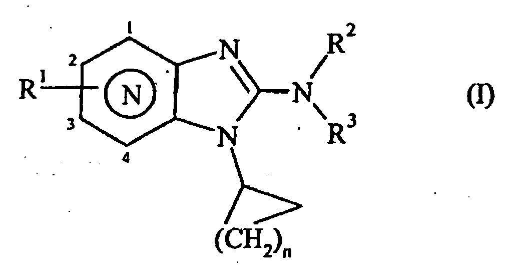 in der:
◆ R¹ ein Wasserstoffatom, ein Halogenatom, eine Alkyl-, Polyhalogenalkyl-, Cyano-, Nitro-, Hydroxycarbonyl-, Alkoxycarbonyl-, Aminocarbonyl-, Alkylaminocarbonyl- oder Dialkylaminocarbonyl-gruppe bedeutet,
◆ R² ein Wasserstoffatom, eine Alkylgruppe, eine gegebenenfalls substituierte Arylgruppe, eine gegebenenfalls substituierte Heteroarylgruppe oder eine Gruppe R²⁰-C(X)- bedeutet, worin:
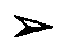 R²⁰ eine Alkylgruppe, Alkoxygruppe, Aminogruppe, Alkylaminogruppe, Dialkylaminogruppe, gegebenenfalls substituierte Arylgruppe oder gegebenenfalls substituierte Heteroarylgruppe und
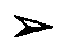 X ein Sauerstoffatom, ein Schwefelatom oder eine Gruppe NR²¹, worin R²¹ ein Wasserstoffatom oder eine Alkylgruppe darstellt, bedeuten,
◆ R³ ein Wasserstoffatom oder eine Alkylgruppe bedeutet,
◆ n eine ganze Zahl mit einem Wert zwischen 1 und 6 einschließlich bedeutet,
◆ der Rest 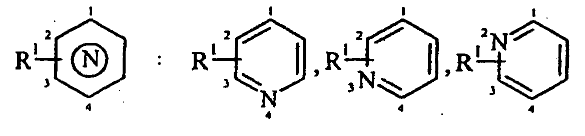 oder 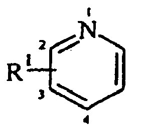 darstellt,
deren Enantiomere, Diastereoisomere sowie deren Additionssalze mit einer pharmazeutisch annehmbaren Säure oder Base,
wobei es sich versteht, daß:
- der Begriff "Alkyl" für eine geradkettige oder verzweigte Kohlenwasserstoffkette steht, die 1 bis 6 Kohlenstoffatome enthält,
- der Begriff "Alkoxy" für eine Alkyloxygruppe steht, deren geradkettige oder verzweigte Alkylkette 1 bis 6 Kohlenstoffatome enthält,
- der Begriff "Aryl" für eine Phenyl- oder Biphenyl-gruppe steht,
- der Begriff "Polyhalogenalkyl" für eine geradkettige oder verzweigte Kohlenstoffkette steht, die 1 bis 3 Kohlenstoffatome und 1 bis 7 Halogenatome enthält,
- der Begriff "Heteroaryl" für eine monocyclische oder bicyclische Gruppe mit 5 bis 11 Kettengliedern steht, bei der mindestens einer der Ringe aromatisch ist, und die in dem Monocyclus oder dem Bicyclus 1, 2 oder 3 Heteroatome ausgewählt aus Stickstoff, Sauerstoff und Schwefel enthält, und
- der Begriff "gegebenenfalls substituiert" im Hinblick auf die Begriffe Aryl und Heteroaryl bedeutet, daß die betreffenden Gruppen durch eine oder zwei gleichartige oder verschiedenartige Gruppen gegebenenfalls substituiert sind, ausgewählt aus Halogenatomen und Alkyl-, Alkoxy-, Polyhalogenalkyl-, Hydroxy-, Cyano-, Nitro-, Amino- (gegebenenfalls durch eine oder zwei Alkylgruppen substituiert) und -C(O)R_{d}, worin R_{d} eine Gruppe bedeutet ausgewählt aus Hydroxy, Alkoxy und Amino, mit der Maßgabe, daß die Heteroarylgruppe zusätzlich am nicht-aromatischen Teil der Heteroarylgruppe durch eine Oxogruppe substituiert sein kann.

2. Verbindungen der Formel (I) nach Anspruch 1, worin der Rest 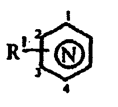 der Gruppe 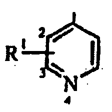 entspricht, deren Enantiomere, Diastereoisomere sowie deren Additionssalze mit einer pharmazeutisch annehmbaren Säure oder Base.

3. Verbindungen der Formel (I) nach einem der Ansprüche 1 oder 2, worin R¹ ein Wasserstoffatom bedeutet, deren Enantiomere, Diastereoisomere sowie deren Additionssalze mit einer pharmazeutisch annehmbaren Säure oder Base.

4. Verbindungen der Formel (I) nach einem der Ansprüche 1 bis 3, worin R² ein Wasserstoffatom bedeutet, deren Enantiomere, Diastereoisomere sowie deren Additionssalze mit einer pharmazeutisch annehmbaren Säure oder Base.

5. Verbindungen der Formel (I) nach einem der Ansprüche 1 bis 3, worin R² eine Gruppe R²⁰-C(O)- bedeutet, deren Enantiomere, Diastereoisomere sowie deren Additionssalze mit einer pharmazeutisch annehmbaren Säure oder Base.

6. Verbindungen der Formel (I) nach einem der Ansprüche 1 bis 5, worin R³ ein Wasserstoffatom bedeutet, deren Enantiomere, Diastereoisomere sowie deren Additionssalze mit einer pharmazeutisch annehmbaren Säure oder Base.

7. Verbindungen der Formel (I) nach einem der Ansprüche 1 bis 3, 5 oder 6, worin R²⁰ eine Alkoxygruppe bedeutet, deren Enantiomere, Diastereoisomere sowie deren Additionssalze mit einer pharmazeutisch annehmbaren Säure oder Base.

8. Verbindungen der Formel (I) nach einem der Ansprüche 1 bis 7, worin n eine ganze Zahl zwischen 4 und 6 einschließlich bedeutet, deren Enantiomere, Diastereoisomere sowie deren Additionssalze mit einer pharmazeutisch annehmbaren Säure oder Base.

9. Verbindungen der Formel (I) nach einem der Ansprüche 1 bis 8, worin der Rest 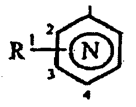 für 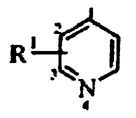 steht, worin R¹ ein Wasserstoffatom bedeutet, R² ein Wasserstoffatom oder eine Gruppe R²⁰-C(O)- darstellt, worin R²⁰ eine Alkoxygruppe bedeutet und n den Wert 4 oder 5 besitzt, deren Enantiomere, Diastereoisomere sowie deren Additionssalze mit einer pharmazeutisch annehmbaren Säure oder Base.

10. Verbindungen der Formel (I) nach einem der Ansprüche 1 bis 4, 6, 8 oder 9, nämlich 3-Cycloheptyl-3*H*-imidazo[4,5-*b*]pyridin-2-amin.

11. Verbindungen der Formel (I) nach einem der Ansprüche 1 bis 4, 6 oder 8, nämlich 3-Cyclooctyl-3*H*-imidazo[4,5-*b*]pyridin-2-amin.

12. Verfahren zur Herstellung der Verbindungen der Formel (I) nach Anspruch 1, **dadurch gekennzeichnet, daß** man als Ausgangsprodukt eine Verbindung der Formel (II) verwendet: 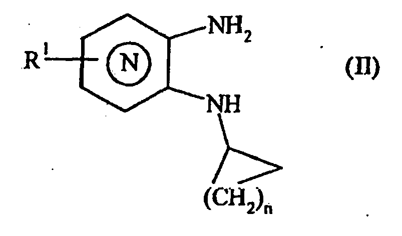 in der R¹ und n die bezüglich der Formel (I) angegebenen Bedeutungen besitzen, welche Verbindungen der Formel (II) mit den Isothiocyanat-Derivaten (III) kondensiert werden:
S = C = N - C(X)-R²⁰ (III)
in der X und R²⁰ die bezüglich der Formel (I) angegebenen Bedeutungen besitzen, zur Bildung Zwischenprodukte der Formel (IV): 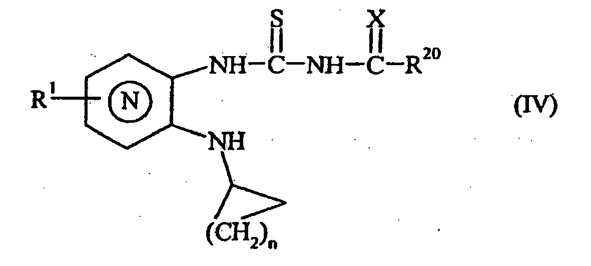 in der R¹, n, X und R²⁰ die bezüglich der Formel (I) angegebenen Bedeutungen besitzen,
welche Verbindungen der Formel (IV) einer intramolekularen Cyclisierung in basischem Medium in Gegenwart eines geeigneten Katalysators unterworfen werden zur Bildung der Verbindungen (I/a): 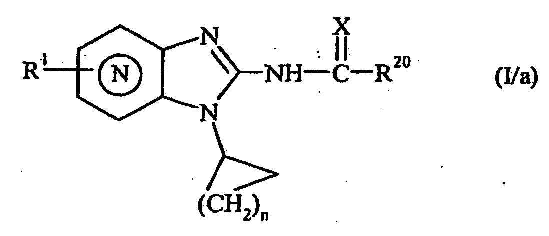 einem Sonderfall der Verbindungen der Formel (I), worin R¹, n, X und R²⁰ die bezüglich der Formel (I) angegebenen Bedeutungen besitzen,
welche Verbindungen der Formel (I/a) gegebenenfalls in saurem Medium in die Verbindungen der Formel (I/b) überführt werden: 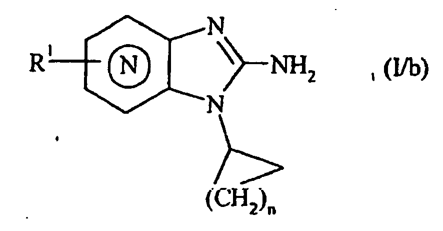 einem Sonderfall der Verbindungen der Formel (I), worin R¹ und n die bezüglich der Formel (I) angegebenen Bedeutungen besitzen,
von welchen Verbindungen der Formel (I/b) die Aminfunktion in basischem Medium mit einem Alkylhalogenid Alk-Z (worin Alk eine Alkylgruppe und Z ein Halogenatom bedeuten) funktionalisiert werden kann zur Bildung der Verbindungen der Formel (I/c): 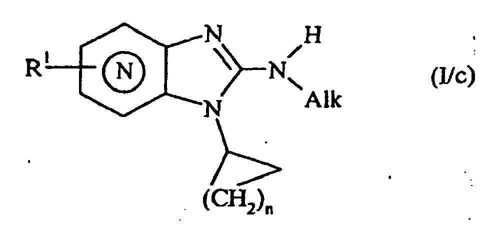 einem Sonderfall der Verbindungen der Formel (I), worin R¹ und n die bezüglich der Formel (I) angegebenen Bedeutungen besitzen und Alk die oben angegebenen Bedeutungen aufweist,
welche Verbindungen der Formeln (I/b) und (I/c) gegebenenfalls in basischem Medium in Gegenwart geeigneter Katalysatoren mit R² - Z' (worin R² die bezüglich der Formel (I) angegebenen Bedeutungen besitzt und Z' eine nucleofuge Gruppe, wie ein Halogenatom oder eine Trihalogenalkylgruppe darstellt) umgesetzt werden können zur Bildung der Verbindungen der Formel (I),
wobei die Verbindungen (I/a), (I/b) und (I/c), welche die Gesamtheit der Verbindungen der Formel (I) bilden,
- gegebenenfalls mit Hilfe einer klassischen Reinigungsmethode gereinigt werden können,
- man gegebenenfalls mit Hilfe einer klassischen Trenntechnik in ihre Stereoisomeren auftrennt und
- man gewünschtenfalls in ihre Additionssalze mit einer pharmazeutisch annehmbaren Säure oder Base überführt,
mit der Maßgabe:
- daß man zu irgendeinem geeigneten Zeitpunkt im Verlaufe des oben beschriebenen Verfahrens die Carbonyl-, Amino-, Alkylamino-gruppe(n) der Ausgangsreaktionsteilnehmer (II) und (III) geschützt werden können und nach der Kondensation zum Zwecke der Synthese wieder von den Schutzgruppen befreit werden können,
- und die Reaktionsteilnehmer (II) und (III) mit Hilfe in Literatur beschriebener bekannter Verfahren hergestellt werden.

13. Pharmazeutische Zubereitung, enthaltend als Wirkstoff mindestens eine Verbindung nach einem der Ansprüche 1 bis 11 allein oder in Kombination mit einem oder mehreren inerten, nichttoxischen und pharmazeutisch annehmbaren Trägermaterialien oder Hilfsstoffen.

14. Pharmazeutische Zubereitung nach Anspruch 13, enthaltend mindestens einen Wirkstoff nach einem der Ansprüche 1 bis 11, für die Herstellung von Arzneimitteln, die als AMPK-Aktivatoren nützlich sind.

15. Pharmazeutische Zubereitung nach Anspruch 13, enthaltend mindestens einen Wirkstoff nach einem der Ansprüche 1 bis 11, nützlich für die Herstellung von Arzneimitteln zur Behandlung des nicht-insulinabhängigen Diabetes Typ II, der Fettsucht, des Diabetes Typ I, der Hyperlipidämie, der Hypercholesterolämie und deren kardiovaskulären Komplikationen.

16. Pharmazeutische Zubereitung nach Anspruch 13, enthaltend mindestens einen Wirkstoff nach einem der Ansprüche 1 bis 11, nützlich für die Herstellung von Arzneimitteln zur Behandlung des Diabetes Typ I, Typ 11 und ihre kardiovaskulären Komplikationen.

17. Pharmazeutische Zubereitung nach Anspruch 13, enthaltend mindestens einen Wirkstoff nach einem der Ansprüche 1 bis 11, nützlich für die Herstellung von Arzneimitteln zur Behandlung des Diabetes Typ I und II.
